(19) Europäisches Patentamt
European Patent Office
Office européen des brevets

(11) Publication number: **0 121 344 A2**

(12) # EUROPEAN PATENT APPLICATION

(21) Application number: **84301404.4**

(22) Date of filing: **02.03.84**

(51) Int. Cl.³: **C 07 D 249/08**
C 07 D 233/56, A 01 N 47/38

(30) Priority: **03.03.83 JP 33846/83**

(43) Date of publication of application:
**10.10.84 Bulletin 84/41**

(84) Designated Contracting States:
**CH DE FR GB LI**

(71) Applicant: **NIPPON KAYAKU KABUSHIKI KAISHA**
**Tokyo Fujimi Bldg. 11-2 Fujimi 1-chome Chiyoda-ku**
**Tokyo102(JP)**

(72) Inventor: **Nakagawa, Taizo**
**606 Koshikiya**
**Ageo-shi Saitama-ken(JP)**

(72) Inventor: **Tejima, Iwao**
**633-3 Haraichi**
**Ageo-shi Saitama-ken(JP)**

(72) Inventor: **Koike, Kengo**
**493-3 Jitoukata**
**Ageo-shi Saitama-ken(JP)**

(72) Inventor: **Tanaka, Eiichi**
**1090 Kami-Ochiai**
**Yono-shi Saitama-ken(JP)**

(72) Inventor: **Matsumoto, Shozo**
**3-439 Miyahara-cho**
**Ohmiya-shi Saitama-ken(JP)**

(72) Inventor: **Fukunaga, Katsuhisa**
**606 Koshikiya**
**Ageo-shi Saitama-ken(JP)**

(74) Representative: **Woods, Geoffrey Corlett et al,**
**J.A. KEMP & CO. 14 South Square Gray's Inn**
**London WC1R 5EU(GB)**

(54) **N,N-substituted azolecarboxamide derivatives useful as fungicides and nematicides.**

(57) N,N-substituted azolecarboxamide derivatives represented by the formula (I):

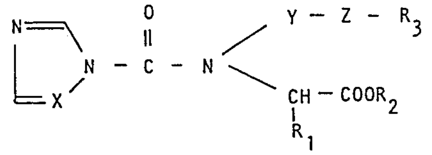

(I)

wherein $R_1$ is hydrogen or lower alkyl, $R_2$ is lower alkyl unsubstituted or substituted by lower alkoxy, $R_3$ is lower alkyl, X is a carbon or nitrogen atom, Y is lower alkylene unsubstituted or substituted by lower alkyl and Z is oxygen or sulfur; are useful as fungicides and nematicides.

EP 0 121 344 A2

# N,N-SUBSTITUTED AZOLECARBOXAMIDE DERIVATIVES USEFUL AS FUNGICIDES AND NEMATICIDES

The present invention relates to N,N-substituted azolecarboxamide derivatives, their preparation, fungicidal and nematicidal compositions for agriculture or horticulture containing them and methods for combating or preventing diseases caused by fungi and for controlling nematodes using them.

Hitherto, as fungicides used for protecting agricultural and horticultural plants from disease, organochlorine compounds, organosulfur compounds and gaseous compounds have been known and utilized. However, organochlorine compounds have the demerits of frequently being phytotoxic to crops and of frequently remaining in plant bodies and in soil due to their use in high concentrations because of their low effectiveness. Organosulfur compounds have the demerits of frequently being phytotoxic to crops and of causing contact dermatitis to a person handling them. In addition, gaseous compounds have the demerits of an irritating and unpleasant odor. As nematicides, there have been used so-called fumigants. However, the fumigants have the demerits of frequently being phytotoxic to crops, of having an irritating or unpleasant odor and of not being applicable directly onto plants.

The present inventors have found that the compounds represented by formula (I) below show a noticeable preventative effect in relation to crop diseases and nematodes, for instance Fusarium wilt, powdery mildew, scab, grey mold, rice blast, sheath blight of rice, brown spot of rice, Verticillium wilt, yellows, damping off, storage

diseases of citrus, seed diseases, rice white-tip nematode, chrysanthemum folia nematode, root-knot nematodes, lesion nematodes, cyst nematodes, pine wood nematode, etc., without showing the disadvantages of the fungicides, bactericides and nematocides hitherto known and used. Moreover, quite unexpectedly they exhibit a noticeable preventative effect on diseases of crop plants, for instance powdery mildew, scab, grey mold, etc., when applied to the soil where the crop plants have been grown, without showing bad effects such as phytotoxicity to the crop plants.

The present invention therefore provides compounds (hereinafter referred to as the present compound) represented by the formula:

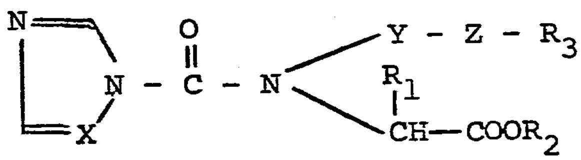

(I)

wherein $R_1$ is hydrogen or lower alkyl, $R_2$ is lower alkyl unsubstituted or substituted by lower alkoxy, for example methoxyethyl, $R_3$ is lower alkyl, X is carbon or nitrogen, Y is lower alkylene unsubstituted or substituted by lower alkyl and Z is oxygen or sulfur. Herein, lower alkyl, lower alkoxy and lower alkylene groups contain from 1 to 6, preferably from 1 to 4, carbon atoms.

The present compound is synthesized by either process (A) or process (B) below as follows:

In process (A), a carbamoyl halide represented

by the formula (II) is brought into reaction with an azole, preferably in an inert solvent at a temperature of 0 to 100°C for about 10 min to several hours to obtain the present compound:

Imidazole

or

Triazole

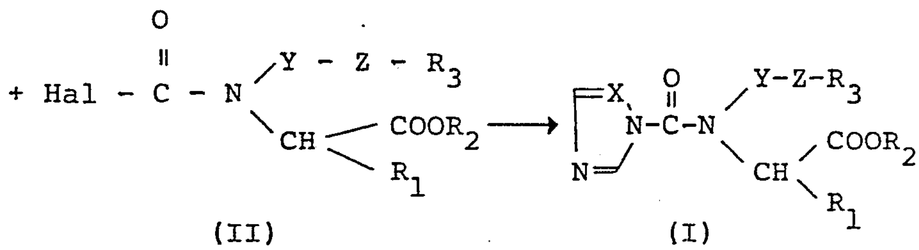

(II) (I)

wherein $R_1$, $R_2$, $R_3$, Y and Z are defined above and Hal represents a halogen atom.

A hydrogen halide generated during the reaction is preferably caught by a tertiary amine such as triethylamine, pyridine and the like or an excess of the azole.

In the process (B), a carbonylbisazole represented by the formula (III) reacts with a secondary amine represented by the formula (IV), preferably in an inert solvent at a temperature of 0 to 100°C for about 10 min to several hours to obtain the present compound as follows:

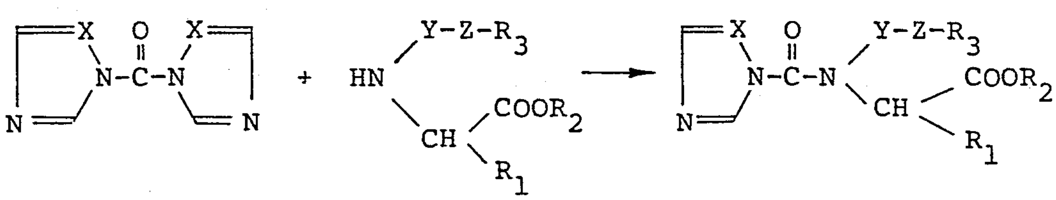

(III) (IV) (I)

wherein $R_1$, $R_2$, $R_3$, Y and Z are defined above.

The inert solvent used in the process (A) or (B) is

an aromatic or aliphatic hydrocarbon or chlorinated aromatic or aliphatic hydrocarbon, for instance, benzene, toluene, xylene, chlorobenzene, dichlorobenzene, dichloromethane, 1,1,1-trichloroethane, chloroform, carbon tetrachloride, and the like or an ether, for instance, diethyl ether, dioxane and tetrahydrofuran.

In addition, a carbamoyl halide represented by the formula (II) can be obtained by reacting a secondary amine represented by the formula (IV) with a carbonyl halide represented by the formula (V) or (VI) in an inert solvent at a temperature of 0 to 100°C for about 10 min to several hours:

$$CO(Hal)_2 \qquad (V)$$

$$(Hal)_3C-O-\overset{\overset{O}{\|}}{C}-Hal \qquad (VI)$$

wherein Hal is defined above.

A carbonylbisazole represented by the formula (III) can be obtained by reacting an azole with a carbonyl halide represented by the formula (V) or (VI) in an inert solvent at a temperature of 0 to 100°C for about 10 min to several hours, preferably, in the presence of a suitable acid-binding agent, for instance, a tertiary amine such as triethylamine and pyridine.

As a carbonyl halide represented by the formula (V), phosgene is preferable, and as a compound represented by the formula (VI), trichloromethyl chloroformate is preferable.

Imidazole or triazole used in the present invention can be produced by a publicly known process. In addition, a secondary amine represented by the formula (IV) are produced by a process wherein an ester of halogenocarboxylic acid represented by the formula:

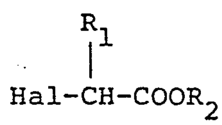

(VII)

wherein $R_1$ and $R_2$ are the same as defined in the formula (I) and Hal is a halogen atom reacts with an amine represented by the formula:

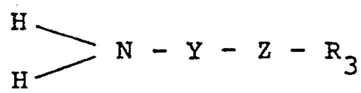

(VIII)

wherein $R_3$, Y and Z are the same as in the formula (I).

As a lower alkyl in the formula (I), methyl, ethyl, propyl, isopropyl, butyl, isobutyl group or secbutyl may be mentioned.

As a halogen atom represented by Hal in the formula (II), a chlorine atom and bromine atom may be mentioned.

The representative compounds according to the present invention, which are produced in the above-mentioned processes, are shown in Table 1.

Table 1

| Compound No. | Structural formula | M.P. or refractive index | Appearance |
|---|---|---|---|
| 1 | 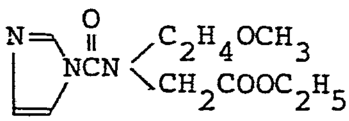 | m.p. 69-71°C | Pale yellow crystal |
| 2 | 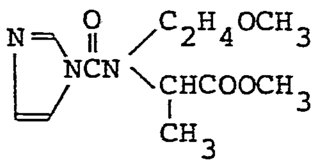 | $n_D^{25}$ 1.4957 | Pale yellow oil |
| 3 | 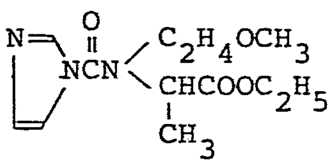 | $n_D^{25}$ 1.4900 | " |
| 4 | 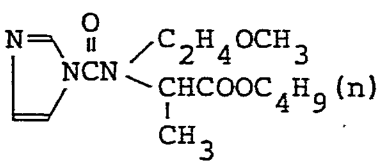 | $n_D^{25}$ 1.4802 | " |
| 5 | 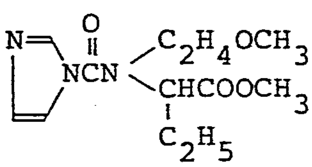 | $n_D^{25}$ 1.4940 | " |
| 6 | 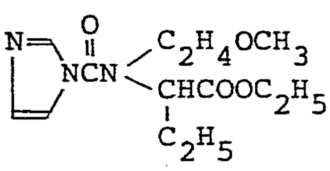 | $n_D^{25}$ 1.4872 | " |
| 7 | 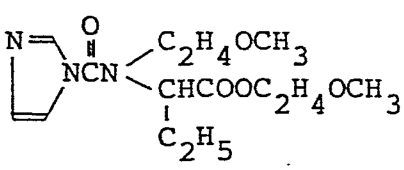 | $n_D^{25}$ 1.4901 | " |
| 8 | 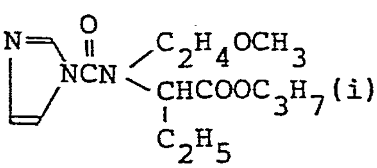 | $n_D^{25}$ 1.4826 | " |

Table 1 (cont'd)

| Compound No. | Structural formula | M.P. or refractive index | Apperance |
|---|---|---|---|
| 9 | 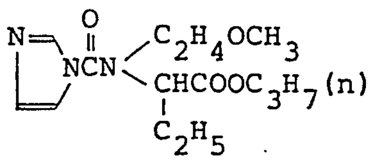 | $n_D^{25}$ 1.4855 | Pale yellow oil |
| 10 | 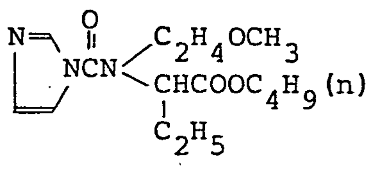 | $n_D^{25}$ 1.4856 | " |
| 11 | 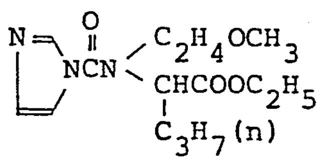 | $n_D^{25}$ 1.4865 | " |
| 12 | 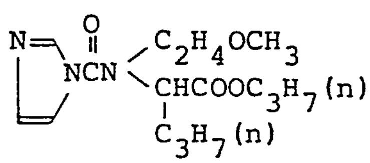 | $n_D^{25}$ 1.4852 | " |
| 13 | 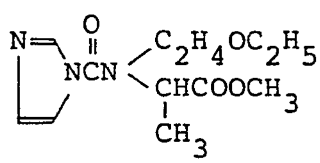 | $n_D^{25}$ 1.4903 | Pale brown oil |
| 14 | 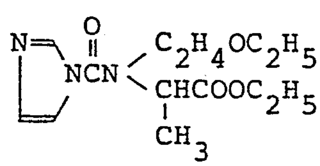 | $n_D^{25}$ 1.4846 | Pale yellow oil |
| 15 | 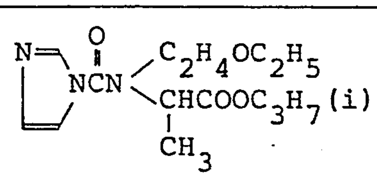 | $n_D^{25}$ 1.4793 | " |

Table 1 (cont'd)

| Compound No. | Structural formula | M.P. or refractive index | Appearance |
|---|---|---|---|
| 16 | 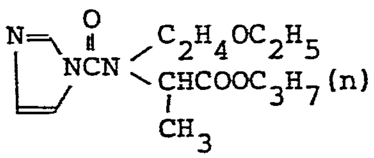 | $n_D^{25}$ 1.4842 | Pale yellow oil |
| 17 | 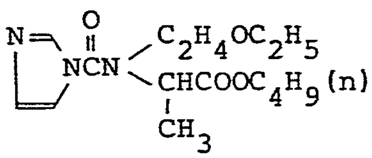 | $n_D^{25}$ 1.4822 | " |
| 18 | 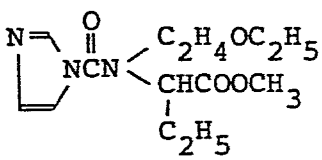 | $n_D^{25}$ 1.4881 | " |
| 19 | 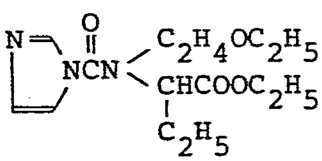 | $n_D^{25}$ 1.4820 | " |
| 20 | 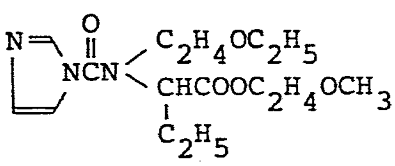 | $n_D^{25}$ 1.4840 | " |
| 21 | 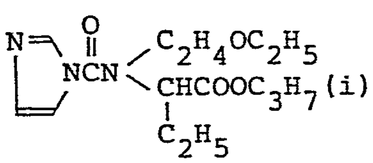 | $n_D^{25}$ 1.4788 | " |
| 22 | 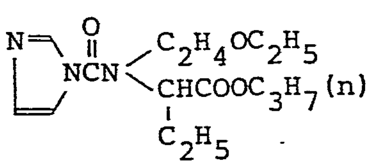 | $n_D^{25}$ 1.4824 | " |

Table 1 (cont'd)

| Compound No. | Structural formula | M.P. or refractive index | Appearance |
|---|---|---|---|
| 23 | 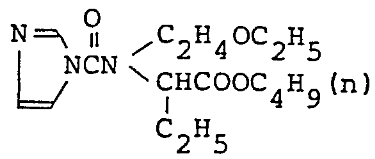 | $n_D^{25}$ 1.4820 | Pale yellow oil |
| 24 | 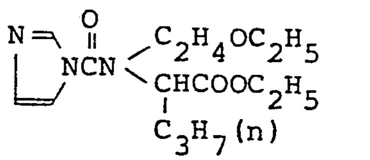 | $n_D^{25}$ 1.4826 | " |
| 25 | 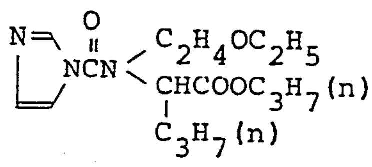 | $n_D^{25}$ 1.4814 | " |
| 26 | 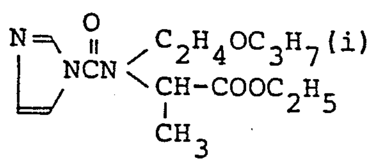 | $n_D^{25}$ 1.4820 | " |
| 27 | 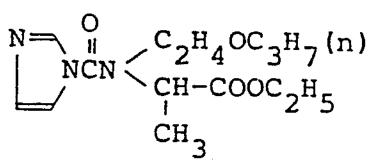 | $n_D^{25}$ 1.4838 | Pale brown oil |
| 28 | 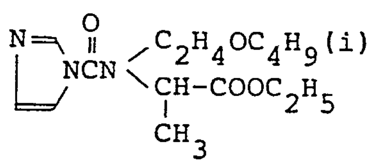 | $n_D^{25}$ 1.4807 | " |
| 29 | 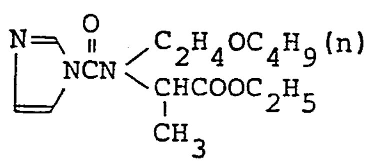 | $n_D^{25}$ 1.4822 | " |
| 30 | 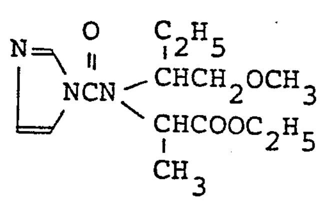 | $n_D^{25}$ 1.4898 | Pale yellow oil |

Table 1 (cont'd)

| Compound No. | Structural formula | M.P. or refractive index | Appearance |
|---|---|---|---|
| 31 | 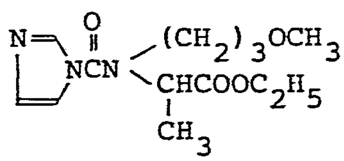 | $n_D^{25}$ 1.4869 | Pale brown oil |
| 32 | 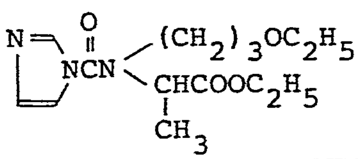 | $n_D^{25}$ 1.4855 | " |
| 33 | 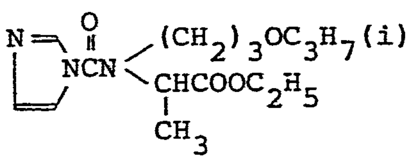 | $n_D^{25}$ 1.4798 | " |
| 34 | 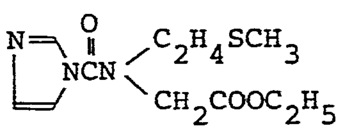 | $n_D^{25}$ 1.5222 | Pale yellow oil |
| 35 | 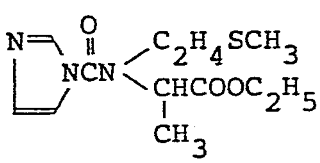 | $n_D^{25}$ 1.5170 | " |
| 36 | 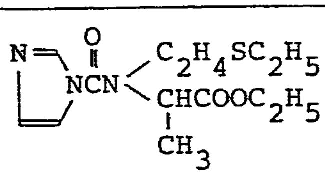 | $n_D^{25}$ 1.5080 | " |
| 37 | 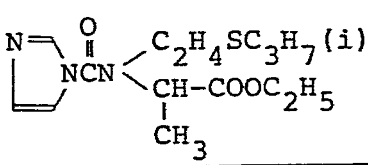 | $n_D^{25}$ 1.5095 | " |
| 38 | 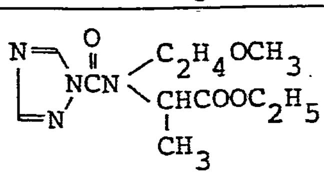 | $n_D^{25}$ 1.4815 | " |
| 39 | 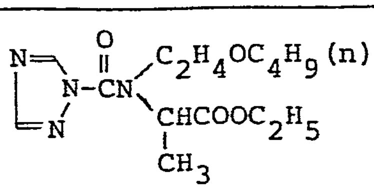 | $n_D^{25}$ 1.4749 | " |

Among the compounds of formula (I), the preferable compounds are those wherein $R_1$ is methyl or ethyl, $R_2$ is lower alkyl, $R_3$ is methyl or ethyl, X is carbon, Y is ethylene and Z is oxygen.

The more preferable compounds are those of formula (I) wherein $R_1$ is methyl or ethyl, $R_2$ is methyl, ethyl or propyl, $R_3$ is methyl or ethyl, X is carbon, Y is ethylene and Z is oxygen.

The most preferable compounds are those of formula (I) wherein $R_1$ is ethyl, $R_2$ is ethyl, n-propyl or i-propyl, $R_3$ is ethyl, X is carbon and Y is ethylene.

The process for producing the present compound is illustrated by the following examples:

Synthesis Example 1:

N-(2-methoxyethyl)-N-(1-n-butoxycarbonylethyl)-imidazole-1-carboxamide (Compound No.4).

4.9g (0.025 mol) of trichloromethylchloroformate were added dropwise to 150 ml of ethylacetate containing 10.2 g (0.05 mol) of N-(2-methoxyethyl)-alanine-n-butyl ester, while keeping the reaction temperature at 0 - 10°C. The mixture was stirred for 1 hour at room temperature. Then 6.8 g of (0.1 mol) of imidazole and 4.0 g (0.05 mol) of pyridine were added to the mixture. The whole was refluxed for 4 hours and cooled to room temperature and then poured into water.

The oil phase was washed with water containing sodium chloride, dried over anhydrous sodium sulfate, subjected to

filtration, and then concentrated. The obtained crude oil was purified with silica gel column chromatography and 10.1 g (0.034 mol) of pale yellow oil was obtained. The yield was 67.9 %.

The physical constants of the compound were as follows:

Refractive index : $n_D^{25}$ 1.4802

Infrared absorption spectrum:

1680 $cm^{-1}$(amide linkage), 1730 $cm^{-1}$(ester linkage)

Nuclear magnetic resonance spectrum(100MHz, TMS, $CCl_4$):

6.88 ppm (s, 1H) ⎫
7.24 ppm (s, 1H) ⎬ imidazole ring
7.80 ppm (s, 1H) ⎭

3.30 ppm (s, 3H) $>N-CH_2CH_2OC\underline{H_3}$

1.49 ppm (d, 3H) $>N-CH-CO_2-$
$\quad\quad\quad\quad\quad\quad\quad\quad\quad\quad | $
$\quad\quad\quad\quad\quad\quad\quad\quad\quad C\underline{H_3}$

N-(2-methoxyethyl)-alanine-n-butyl ester which is an intermediate of Compound No. 4 was produced as follows:

20.9 g (0.1 mol) of α-bromo propionic acid n-butyl ester was added dropwise to the mixed solution of 7.5 g (0.1 mol) of 2-methoxyethylamine and 11.1 g (0.11 mol) of triethylamine. Then the reaction was effected 2 hours at room temperature and 2 hours at 50 to 60°C, and the reaction mixture was cooled to room temperature and then 50 ml of water were added thereto.

The oil was extracted with toluene and the toluene solution was washed with water, dried over anhydrous sodium sulfate, subjected to filtration and concentrated.

The obtained substance was distilled under reduced pressure to obtain 16.9 g (0.083 mol) of colorless transparent oil having a boiling point of 126 - 128°C/18 mmHg.

The yield was 83 %.

Synthesis Example 2:

N-(1-ethoxycarbonylethyl)-N-(2-ethoxyethyl)-imidazole-1-carboxamide (Compound No. 14).

25.7 g (0.13 mol) of trichloromethylchloroformate was added dropwise to 300 ml of ethyl acetate containing 17.7 g (0.26 mol) of imidazole and 26.3 g (0.26 mol) of triethylamine, while keeping the reaction temperature at 0 - 10°C and the mixture was stirred for 1 hour.

The mixture of 37.9 g (0.2 mol) of N-(2-ethoxyethyl) alanine ethyl ester and 20.2 g (0.2 mol) of triethylamine was added to the above mixture at 0 - 10°C.

Then the whole was refluxed for 3 hours, cooled to room temperature and poured into water.

The oil phase was washed with water containing sodium chloride, dried over anhydrous sodium sulfate, subjected to filtration and concentrated. The obtained crude oil was purified with silica gel column chromatography and 40.9 g (0.144 mol) of pale yellow oil was obtained. The yield was 72.0 %.

The physical constants of the compound were as follows:

Refractive index : $n_D^{25}$ 1.4846

Infrared absorption spectrum:

1690 $cm^{-1}$(amide linkage), 1735 $cm^{-1}$(ester linkage)

Nuclear magnetic resonance spectrum(100MHz, TMS, $CCl_4$)

| | |
|---|---|
| 6.96 ppm (s, 1H) | imidazole ring |
| 7.36 ppm (s, 1H) | imidazole ring |
| 7.92 ppm (s, 1H) | imidazole ring |
| 1.52 ppm (d, 3H) | $>N-CH(\underline{CH_3})-CO_2-$ |

N-(2-ethoxyethyl)alanine ethyl ester which is an intermediate of Compound No. 14 was produced as follows:

54.3 g (0.3 mol) of α-bromo propionic acid ethyl ester were added dropwise to the mixture of 26.8 g (0.3 mol) of 2-ethoxyethylamine and 33.4 g (0.33 mol) of triethylamine, while keeping the reaction temperature at 40 to 60°C by cooling with water bath.

After the reaction was effected at room temperature for 2 hours and at 50 to 60°C for 2 hours, the reaction mixture was cooled to room temperature and 150 ml of water was added thereto.

The oil was extracted with toluene, the toluene solution was washed with water, dried over anhydrous sodium sulfate, subjected to filtration and concentrated. The obtained crude oil was distilled under reduced pressure to obtain 44.9 g (0.237 mol) of colorless transparent oil having a boiling point of 113 to 116°C/21 mmHg. The yield was 79.0%.

Synthesis Example 3:

N-(ethoxycarbonylmethyl)-N-(2-methylthioethyl)-imidazole-1-carboxamide(Compound No. 34).

2.47 g (0.0125 mol) of trichloromethylchloroformate was added dropwise to 60 ml of tetrahydrofuran containing 2.12 g (0.0312 mol) of imidazole and 1.98 g (0.025 mol) of pyridine, while keeping the reaction temperature of 5 to 15°C and the whole was stirred for 1 hour.

To the reaction solution, the mixture of 3.69 g (0.0208 mol) of N-(2-methylthioethyl)glycine ethyl ester and 1.81 g (0.0229 mol) of pyridine were added thereto at 5 to 15°C. Thereafter, the reaction was effected for 5 hours at 40 to 50°C, cooled to room temperature and the reaction mixture was poured to water and extracted with toluene. The toluene solution was washed with water containing sodium chloride, dried over anhydrous sodium sulfate, subjected to filteration and concentrated. The obtained crude oil was purified by silica gel column chromatography to obtain 4.03 g (0.0149 mol) of pale yellow oil. The yield was 71.6 %

The physical constants of the compound were as follows:

Refractive index : $n_D^{25}$ 1.5222

Infrared absorption spectrum:

1690 $cm^{-1}$(amide linkage), 1740 $cm^{-1}$(ester linkage)

Nuclear magnetic resonance spectrum:

| | |
|---|---|
| 6.96 ppm (s, 1H)<br>7.26 ppm (s, 1H)<br>7.86 ppm (s, 1H) | imidazole ring |
| 2.02 ppm (s, 3H) | $-S-CH_3$ |
| 1.24 ppm (t, 3H)<br>4.18 ppm (m, 2H) | $-O-CH_2-CH_3$ |

N-(2-methylthioethyl)glycine ethyl ester which is an intermediate of Compound No. 34 was produced as follows:

11.0 g (0.09 mol) of chloroacetic acid ethyl ester were added dropwise to the solution of 9.1 g (0.1 mol) of 2-methylthioethylamine and 10.1 g (0.1 mol) of triethylamine.

The reaction was effected for 4 hours at 50 to 55°C and the whole was poured to water after cooling to room temperature.

The obtained oil was extracted with toluene, dried over anhydrous sodium sulfate, subjected to filtration and concentrated. The crude oil was purified with silica gel column chromatography, distilled under reduced pressure to obtain 10.4 g (0.0587 mol) of pale yellow oil having a boiling point of 112 to 114°C/17 mmHg. The yield was 65.2 %.

In the case where the present compound is used as a fungicide or a nematicide, it is directly used as it is, or is used in a form of compositions such as dusts, micro granule, granules, wettable powders, flowable compositions, emulsifiable concentrates and the like prepared by the usually adopted method in a field of agrochemicals production while being mixed

with at least one adjuvant in order to promote or stabilize the effects thereof.

These various compositions can be directly applied in fields as they are or applied after being diluted with water to a desired concentration.

As an adjuvant herein mentioned, a carrier(diluent) and the other adjuvants such as a sticker, an emulsifier, a wetting agent, a dispersant, a fixing agent and a disintegrator may be mentioned.

As a liquid carrier, an aromatic hydrocarbon such as toluene, xylene, an alcohol such as methanol, butanol, glycol, a ketone such as acetone, an amide such as dimethylformamide, a sulfoxide such as dimethylsulfoxide, and others such as methylnaphthalene, cyclohexane, animal and vegetable oils, fatty acids, esters of fatty acid may be mentioned.

As a solid carrier, clay, kaolin, talc, diatomaceous earth, silica, calcium carbonate, montmorillonite, bentonite, feldspar, quartz, alumina, sawdust and the like may be mentioned.

As an emulsifier or a dispersing agent, usually a surfactant is used and, for instance, anionic surfactants such as sodium higher alcohol sulfates, stearyltrimethyl ammonium chloride, polyoxyethylene alkylphenyl ether, laurylbetain, cationic surfactants, nonionic surfactants and amphoteric surfactants may be mentioned.

In addition, as a sticker, for instance, polyoxyethylene nonyl phenyl ether or polyoxyethylene lauryl ether may

be mentioned, and as a wetting agent, for instance, polyoxyethylene nonyl phenyl ether or dialkyl sulfosuccinate may be mentioned. As a fixing agent, carboxymethylcellulose or polyvinyl alcohol, and as a disintegrator, sodium ligninsulfonate or sodium lauryl sulfate may be mentioned.

Every composition containing the present compound as the active ingredient is not only singly applicable, but also applicable after mixing with other fungicides, insecticides, plant growth regulators, miticides, herbicides, fungicides for soil, soil-improving agents or nematicides and furthermore, it is applicable after mixing with fertilizers.

The content of the present compound as the active ingredient in the fungicidal composition or the nematicidal composition for agricultural or horticultural use depends on the form and shape of the composition, the method for application and other conditions, and although there are cases where the present compound itself is the sole component, the content is usually in a range of 0.5 to 95 % by weight, preferably in the range of 2 to 70 % by weight.

To be more precisely, a preferable range of the content is shown as follows:

| | active ingredient (weight %) | adjuvant (weight %) |
|---|---|---|
| Dust | 1 - 20 | 80 - 99 |
| Emulsion | 2 - 40 | 60 - 98 |
| Wettable powder | 20 - 95 | 5 - 80 |

| | | |
|---|---|---|
| Granule and micro granule | 1 - 20 | 80 - 99 |
| Flowable suspension concentrate | 20 - 80 | 20 - 80 |

The fungicidal composition or nematicidal composition for agricultural or horticultural use according to the present invention is applied on foliage of crop plants or in soil where crop plants are grown, and shows an excellent controlling effects against crop diseases, and particularly, to the diseases on the above-ground part of the crop plants such as powdery mildew, scab, grey mold by application into the soil where the crop plants are grown. In addition, the nematicidal composition according to the present invention shows a remarkable nematicidal effect against rice white-tip nematode, chrysanthemum foliar nematode, root-knot nematodes, lesion nematodes, cyst nematodes, pine wood nematode and the like, and accordingly, can be applied for controlling effectively these nematodes.

In cases of practical application, it is preferable to scatter the composition at a concentration of the active ingredient of 10 to 4,000 ppm onto leaves and stems, and in cases of soil application, the amount is preferably 0.05 to 10 kg per 10 areas.

Furthermore, when the composition of the present invention is used for disinfection of seeds, seeds may be soaked in the solution containing 0.05 - 1.0 % by weight of the active ingredient of the present invention, or 0.1 - 5 % by weight of the active ingredient against the weight of seeds may be used for covering the seeds.

The fungicidal or nematicidal composition for agricultural or horticultural use according to the present invention is illustrated by the following examples, and in these cases, the kinds and the ratio of mixing thereof with the active ingredient are not restricted to the Examples and are changeable in a broad range. In Examples, "part" means part by weight.

FORMULATION EXAMPLE 1:

Dust

Ten parts of Compound No. 3, 41 parts of talc and 49 parts of clay were mixed and by pulverizing the resultant mixture, a dust was prepared.

FORMULATION EXAMPLE 2:

Wettable powder

Eighty parts of Compound No. 14, 3 parts of sodium higher alcohol sulfate and 2 parts of sodium polyacrylate were mixed and by pulverizing the resultant mixture, a wettable powder was prepared.

FORMULATION EXAMPLE 3:

Granule

Three parts of Compound No. 18, 35 parts of diatomaceous earth, 23 parts of bentonite, 37 parts of talc and 2 parts of a disintegrator were mixed and then 18 parts of water was added to the mixture to wet the components uniformly and afterwards the mixture was subjected to an injection molding machine to be extruded as granules. After drying the granules, they were subjected to a crusher and then to a granulator to

obtain a granule of size of 0.6 to 1 mm.

FORMULATION EXAMPLE 4:

Micro granule

Five parts of Compound No. 29, 6 parts of bentonite and 9 parts of clay were uniformly mixed and the resultant mixture was pulverized to be a dense dust composition. Separately, 80 parts of non-oil absorbent coarse mineral powder of 105 - 74 μ was introduced into a suitable mixer and while rotating the mixer, 20 parts of water was introduced into the mixer and after uniformly wetting the mineral matter, the dense dust composition prepared above was introduced into the mixer and then the dense dust composition was covered with the above mineral material to obtain a micro granule.

FORMULATION EXAMPLE 5:

Emulsion

Twenty parts of Compound No. 34 was dissolved in 63 parts of xylene, and 17 parts of a 8:2 mixture of a condensate of alkylphenol and ethylene oxide and calcium alkylbenzene-sulfonate was added to the solution to be dissolved therein to obtain an emulsion.

The excellent effect of the present compound in controlling diseases and nematodes on crop plants of agriculture and horticulture is illustrated by the following experimental examples:

EXPERIMENTAL EXAMPLE 1:

Cucumber powdery mildew-controlling test

Each wettable powder of the compounds of Table 2 was diluted with water to contain each active ingredient at 250 ppm, and was applied onto the potted cucumber seedlings (variety: $F_1$Kyoryoku green fushinari, at the stage of first leaf-developing).

After drying, the cucumber seedlings were inoculated by spraying the spore suspension of Saphaerotheca fuliginea, and after keeping the planted pots in a greenhouse for 2 weeks, the disease degree was investigated then disease index and control value were calculated according to the following formula. As a control, thiophanatemethyl 70 % wettable powder {active ingredient: 1,2-bis(3-methoxycarbonyl-2-thioureide)benzene} was used after diluting with water at a concentration of 350 ppm.

$$\text{Disease Index} = \frac{(3xA) + (2xB) + (1xC)}{3 \times (A+B+C+D)} \times 100$$

wherein A: number of plants on which severe disease occurrence was observed,

B: number of plants on which considerable disease was observed,

C: number of plants on which slight disease was observed,

D: number of plants without disease.

From the thus recorded index of disease, the control value of each fungicidal specimen was calculated according to the following formula:

$$\text{Control value} = \frac{(\text{Disease Index in the untreated plot}) - (\text{Disease Index in the treated plot})}{(\text{Disease Index in the untreated plot})}$$

The results of the test are shown in Table 2.

Table 2

Test results of cucumber powdery mildew-controlling

| Tested compound | | Concentration of active ingredient | Control value | Phytotoxicity |
|---|---|---|---|---|
| 1 | | 250 ppm | 70 | no |
| 2 | | 250 | 100 | no |
| 3 | | 250 | 100 | no |
| 4 | | 250 | 100 | no |
| 5 | | 250 | 100 | no |
| 6 | | 250 | 100 | no |
| 8 | | 250 | 100 | no |
| 9 | | 250 | 100 | no |
| 10 | | 250 | 100 | no |
| 13 | | 250 | 62 | no |
| 14 | | 250 | 100 | no |
| 18 | | 250 | 100 | no |
| 19 | | 250 | 100 | no |
| 21 | | 250 | 100 | no |
| 22 | | 250 | 100 | no |
| 23 | | 250 | 100 | no |
| 26 | | 250 | 100 | no |
| 27 | | 250 | 100 | no |
| 28 | | 250 | 100 | no |
| 29 | | 250 | 100 | no |
| 30 | | 250 | 100 | no |
| 31 | | 250 | 82 | no |
| 32 | | 250 | 76 | no |
| 33 | | 250 | 54 | no |
| 34 | | 250 | 100 | no |
| 35 | | 250 | 80 | no |
| 36 | | 250 | 100 | no |
| 37 | | 250 | 100 | no |
| control | Thiophanate-methyl 70% wettable powder | 350 ppm | 40 | no |
| control | un-treated | - | 0 | - |

EXPERIMENTAL EXAMPLE 2:

Cucumber powdery mildew-controlling test by soil treatment

The seedlings of cucumber (variety: Tokiwa jibai, at the stage of first leaf developing) grown on the potted soil in a pot of 10 cm in diameter, was drenched with the diluted solution of each of the emulsion of the compounds of Table 3 at a rate of 25 ml per pot(the amount of each active ingredient per pot was 0.0125 g).

As a control, 12.5 % liquid formulation containing (5-butyl-2-dimethylamino-6-methylpyrimidine-4-ol) was used.

After three days of the soil treatment, the seedlings were inoculated by spores of *Sphaerotheca fuliginea*, and after keeping the pots in a greenhouse for 2 weeks, disease degree on the seedlings was investigated and the control value of each active-ingredient was calculated according to the formulae in EXPERIMENTAL EXAMPLE 1. The results are shown in Table 3.

Table 3

Test results of cucumber powdery mildew-controlling by soil treatment

| Tested compound | Concentration of active ingredient | Control value | Phytotoxicity |
|---|---|---|---|
| 1 | 12.5 mg/pot | 100 | no |
| 2 | 12.5 | 100 | no |
| 3 | 12.5 | 100 | no |
| 4 | 12.5 | 100 | no |
| 5 | 12.5 | 100 | no |
| 6 | 12.5 | 100 | no |
| 8 | 12.5 | 100 | no |
| 9 | 12.5 | 100 | no |
| 10 | 12.5 | 100 | no |
| 13 | 12.5 | 100 | no |
| 14 | 12.5 | 100 | no |
| 18 | 12.5 | 100 | no |
| 19 | 12.5 | 100 | no |
| 21 | 12.5 | 100 | no |
| 22 | 12.5 | 100 | no |
| 23 | 12.5 | 100 | no |
| 26 | 12.5 | 100 | no |
| 28 | 12.5 | 82 | no |
| 29 | 12.5 | 100 | no |
| 30 | 12.5 | 100 | no |
| 31 | 12.5 | 100 | no |
| 32 | 12.5 | 64 | no |
| 33 | 12.5 | 72 | no |
| 34 | 12.5 | 56 | no |
| 35 | 12.5 | 100 | no |
| 36 | 12.5 | 92 | no |
| 37 | 12.5 | 90 | no |
| Control: Dimethyrimol 12.5% liquid formulation | 12.5 mg/pot | 50 | no |
| Control: untreated | - | 0 | - |

EXPERIMENTAL EXAMPLE 3:

Cucumber Scab-controlling test

Each of the emulsions of the compounds of Table 4 was diluted with water to contain each active ingredient at 500 ppm, and applied onto the potted cucumber seedlings (variety: Tokiwa jibai, at the stage of first leaf-developing).

After drying, the cucumber seedlings were inoculated by spraying the spore suspension of *Cladosporium cucumerinum*. After keeping the planted pots in a wet chamber at 20°C for 24 hours, the planted pots were kept in a greenhouse. The disease degree on the plants was investigated 7 days after inoculation to calculate the control value of each compound according to the formulae shown in EXPERIMENTAL EXAMPLE 1.

The results of the test are shown in Table 4.

Table 4

Test results of Cucumber Scab-controlling test

| Tested compound | Concentration of active ingredient | Control value | Phytotoxicity |
|---|---|---|---|
| 3 | 500 ppm | 82 | no |
| 14 | 500 | 76 | no |

EXPERIMENTAL EXAMPLE 4:

Kidney bean gray mold-controlling test

Each of the emulsions of the compounds of Table 5 was diluted with water to contain each active ingredient at

500 ppm, and thus obtained solution was applied onto the potted seedlings of kidney bean plant (variety: Shin-edogawa, at the stage of primary leaf-developing).

After drying, a piece of cultured myceria of Botrytis cinerea cut out from the culture by a cork-borer of 5 mm in diameter was placed on the primary leaf, and the thus treated plants were kept at a moist chamber to be infected. After 48 hours of the inoculation, the lesion diameter on the primary leaf was investigated to calculate the control value of each compound according to the following formula.

As a control, Euparen 50 % wettable powder (active ingredient: N'-(dichloro fluoromethylthio)-N,N-dimethyl-N'-phenylsulfamide was used.

$$\text{Control value} = \frac{\text{(Lesion diameter in the untreated plot)} - \text{(Lesion diameter in each treated plot)}}{\text{(Lesion diameter in the untreated plot)}} \times 100$$

The results are shown in Table 5.

Table 5

Test results of kidney bean gray mold-controlling test

| Tested compound | | Concentration of active ingredient | Control value | Phytotoxicity |
|---|---|---|---|---|
| 4 | | 500 ppm | 96 | no |
| 13 | | 500 | 92 | no |
| Control | Euparen 50% wettable powder | 500 ppm | 62 | no |

EXPERIMENTAL EXAMPLE 5:

Controlling test against Verticillium wilt on eggplant

Each of the emulsions of the compounds of Table 6 was diluted with water, and it was applied into the soil in the pot at a rate of 50 ml per pot where eggplant (variety: Senryo No. 2, at the stage of 5 to 6 leaves) had been grown. Afterwards, a spore suspension of *Verticillium albo-atrum* was injected into the stem of the eggplant to inoculate the plant. Ten days after inoculation, the disease index was investigated and the index of disease and the control value were calculated according to the following formulae. As a control, benomyl 50 % wettable powder containing methyl 1-(butylcarbamoyl)-2-benzimidazolecarbamate as the active ingredient was used after diluting with water.

$$\text{Disease Index} = \frac{(4 \times A) + (3 \times B) + (2 \times C) + (1 \times D)}{4 \times (A + B + C + D + E)} \times 100$$

wherein A: number of plants on which lesion was observed on more than 2/3 of the leaves thereof, B: number of plants on which lesion was observed on 1/2 to 2/3 of the leaves thereof, C: number of plants on which lesion was observed on 1/3 to 1/2 of the leaves thereof, D: number of plants on which lesion was observed on less than 1/3 of the leaves thereof and E: number of plants without lesion on the leaves thereof.

$$\text{Control value} = \frac{\begin{pmatrix}\text{Disease Index in} \\ \text{the untreated plot}\end{pmatrix} - \begin{pmatrix}\text{Disease Index in} \\ \text{the treated plot}\end{pmatrix}}{\begin{pmatrix}\text{Disease Index in} \\ \text{the untreated plot}\end{pmatrix}} \times 100$$

The test results are shown in Table 6.

Table 6

Test results against Verticillium wilt on eggplant

| Tested compound | | Concentration of active ingredient | Control value | Phytotoxicity |
|---|---|---|---|---|
| 3 | | 0.02 g/pot | 100 | no |
| 14 | | 0.02 | 100 | no |
| Control | benomyl 50 % wettable powder | 0.02 g/pot | 67 | no |

EXPERIMENTAL EXAMPLE 6:

Controlling test against tomato root-knot nematode

After mixing soil infected by *Meloidogyne incognita* with 0.1 g of each of the 10 % dusts of the compound of Table 7 the mixture was packed in an unglazed pot of 12 cm in diameter. The seeds of tomato(variety: Ponte Rosa) was sown in the potted soil at 15 seeds/pot.

After 40 days of the soil-treatment, the seedlings of tomato were dug out from the potted soil, and the phytotoxicity and the gall index were investigated according to the following formula:

$$\text{gall index} = \frac{(4 \times A) + (3 \times B) + (2 \times C) + D}{4 \times (\text{total number of seedlings})} \times 100$$

wherein A: number of seedlings each having more than 31 galls on the roots thereof, B: number of seedlings each having 21 to 30 galls on the roots thereof, C: number of seedlings each having 11 to 20 root-knots on the roots thereof and D: number of seedlings each having 1 to 10 galls on the roots thereof. The results are shown in Table 7.

Table 7

Test results of controlling tomato root-knot nematode

| Tested compound | Concentration of active ingredient | Gall index | Phytotoxicity |
|---|---|---|---|
| 3 | 0.01 g/pot | 25 | no |
| 14 | 0.01 | 26 | no |
| untreated | - | 90 | - |

CLAIMS

1. A compound represented by the formula:

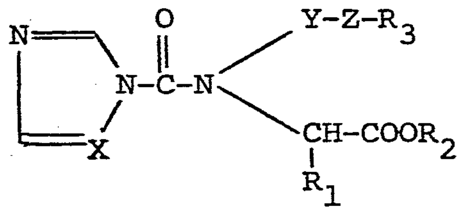

(I)

wherein $R_1$ is hydrogen or lower alkyl, $R_2$ is lower alkyl unsubstituted or substituted by lower alkoxy, $R_3$ is lower alkyl, X is a carbon or nitrogen atom, Y is lower alkylene unsubstituted or substituted by lower alkyl and Z is oxygen or sulfur.

2. A compound according to claim 1 wherein $R_1$ is methyl or ethyl, $R_2$ is unsubstituted lower alkyl, $R_3$ is methyl or ethyl, X is carbon, Y is ethylene and Z is oxygen.

3. A compound according to claim 2 wherein $R_2$ is methyl, ethyl or propyl.

4. A compound according to claim 3 wherein $R_1$ is ethyl, $R_2$ is ethyl, n-propyl or i-propyl, $R_3$ is ethyl, X is carbon and Y is ethylene.

5. A compound according to claim 1 selected from N-(2-methoxyethyl)-N-(1-n-butoxycarbonylethyl)imidazole-1-carboxamide, N-(1-ethoxycarbonylethyl)-N-(2-ethoxyethyl) imidazole-1-carboxamide and N-(ethoxycarbonylmethyl)-N-(2-methylthioethyl)imidazole-1-carboxamide.

6. A process for producing a compound represented by the formula (I) as defined in claim 1, which process comprises reacting a compound of the formula:

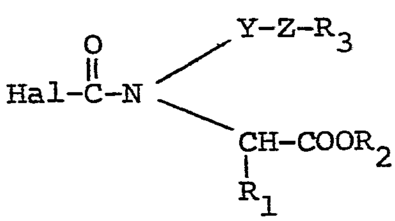

wherein $R_1$, $R_2$, $R_3$, Y and Z are as defined in claim 1 and Hal is halogen, with imidazole or triazole.

7. A process for producing a compound represented by the formula (I) as defined in claim 1, which process comprises reacting a compound represented by the formula:

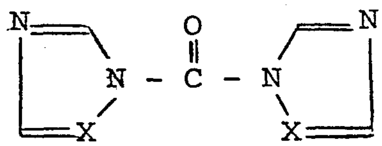

wherein X is as defined in claim 1 with a compound represented by the formula:

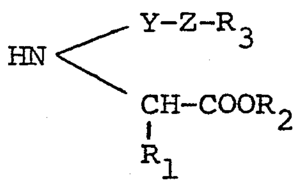

wherein $R_1$, $R_2$, $R_3$, Y and Z are as defined in claim 1.

8. A fungicidal composition which comprises from 0.5 to 95 % by weight of a compound of formula (I) as claimed in any one of claims 1 to 5 and 5 to 99.5 % by weight of a suitable adjuvant therefor.

9. A nematicidal composition which comprises from 0.5 to 95 % by weight of a compound of formula (I) as claimed in any one of claims 1 to 5 and from 5 to 99.5 % by weight of a suitable adjuvant therefor.

10. A method for combating or preventing a disease of agricultural or horticultural plants caused by a fungus at a locus which method comprises applying to said locus a fungicidally effective amount of compound of formula (I) as claimed in any one of claims 1 to 5.

11. A method for controlling nematodes at a locus, which method comprises applying to said locus a nematocidally effective amount of a compound of formula (I) as claimed in any one of claims 1 to 5.

12. A method according to claim 10 or 11 wherein the compound of formula (I) is applied to the earth at said locus.

13. A method according to any one of claims 10 to 12 wherein the compound of formula (I) is applied to plants at said locus.